# EUROPEAN PATENT SPECIFICATION

(11) **EP 2 089 375 B1**
(45) Date of publication and mention of the grant of the patent: **31.07.2013**
(21) Application number: 07846243.9
(22) Date of filing: 05.12.2007
(51) Int. Cl.: C07D 333/20, A61K 31/381, A61P 25/24

(54) **A METHOD OF PURIFICATION OF (S)-N-METHYL-3-(1-NAPHTYLOXY)-3-(2-THIENYL) PROPYLAMINE HYDROCHLORIDE (DULOXETINE)**
VERFAHREN ZUR REINIGUNG VON (S)-N-METHYL-3-(1-NAPHTYLOXY)-3-(2-THIENYL)-PROPYLAMIN-HYDROCHLORID (DULOXETIN)
PROCÉDÉ DE PURIFICATION DU CHLORHYDRATE DE (S)-N-MÉTHYL-3-(1-NAPHTYLOXY)-3-(2-THIÉNYL) PROPYLAMINE (DULOXÉTINE)

(30) Priority: 05.12.2006 CZ 20060773
(43) Date of publication of application: 19.08.2009
(73) Proprietor: Zentiva, k.s., Dolni Mecholupy 102 37 Praha 10 (CZ)
(72) Inventor: RIDVAN, Ludek, 102 00 Praha 10 (CZ); CINIBULK, Josef, 190 00 Praha 9 (CZ); ZATOPKOVA, Monika, 198 00 Praha 9 (CZ); PLACEK, Lukas, 792 01 Bruntal (CZ)
(74) Representative: Jirotkova, Ivana
(86) International application number: PCT/CZ2007/000109
(87) International publication number: WO 2008/067781

(56) References cited:
- WO-A-2004/056795
- WO-A-2005/108386
- WO-A-2006/099433
- GAO LI-MEI ET AL: "Synthesis of Duloxetine hydrochloride" ZHONGGUO XIN YAO ZAZHI - CHINESE NEW DRUGS JOURNAL, GAI-KAN BIANJIBU, BEIJING, CN, vol. 14, no. 1, 1 January 2005 (2005-01-01), pages 74-76, XP009088517 ISSN: 1003-3734

## Description

### Technical Field

The invention deals with a new method of purification of (*S*)-*N*-methyl-3-(1-naphtyloxy)-3-(2-thienyl) propylamine hydrochloride of formula I 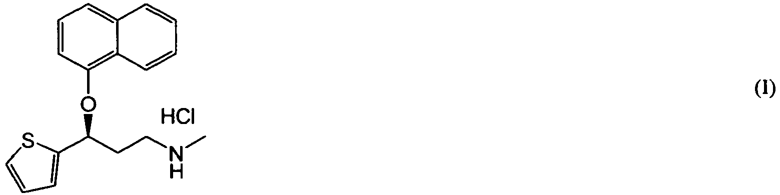 known under the generic name duloxetine.

### Background Art

Duloxetine is a serotonine and noradrenalin reuptake inhibitor and it can be therapeutically used in the sphere of e.g. depression and urinal incontinence. The duloxetine molecule is chiral; (*S*)-enantiomer is used as the effective substance.

Preparation of duloxetine and its intermediate products is described e.g. in the following patents: EP 0 273 658, US 5 362 886, WO 2004/005239, US 2003/0225153. Methods that are commonly used are illustrated in Scheme 1 below.

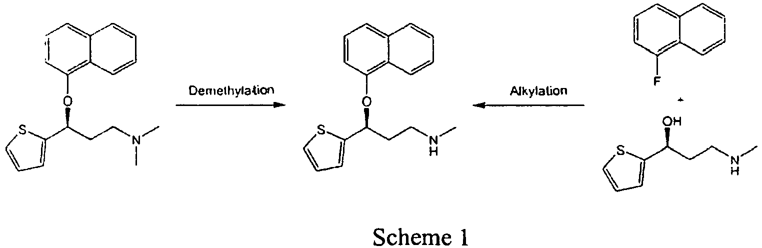

Preparation of a salt of duloxetine is described in Example 2 (preparation 2) of US patent 5 362 886. The final product is achieved through the action of hydrochloric acid upon a solution of duloxetine base in ethyl acetate. A crystal seed of duloxetine hydrochloride is added to the acidified reaction mixture and the mixture is diluted with more ethyl acetate. After 30 minutes' stirring the mixture is concentrated again to the original volume and subsequently it is stirred for 1 hour at the laboratory temperature and for 1 hour at the temperature of 0 °C.
However, when this procedure was reproduced, the obtained duloxetine hydrochloride did not prove to be entirely pure and colourless.

The principal pollutants of duloxetine comprise the (*R*)-enantiomer of formula II and the positional isomer, (*S*)-*N*-methyl-3-(1-naftyloxy)-3-(3-thienyl) propylamine of formula III, which are mentioned in patent applications Nos. WO 2006/099433 and WO 2006/099468.

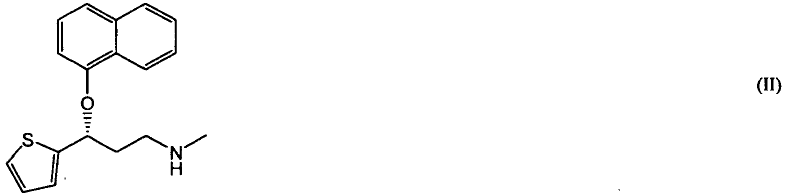

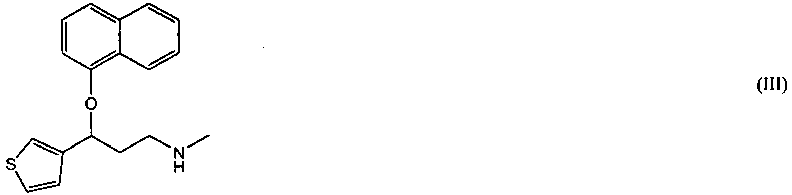

The crude product may contain several other impurities, e.g. products of duloxetine decomposition in an acidic environment (1-naphtol and other). This means that the method of final purification is very important to achieve the required quality and yield of the substance.

Patent documents Nos. WO 2006/045255 and CZ 297560 (both by Zentiva) mention ethyl acetate (EtOAc) and ethylmethylketone (MEK) as the solvent used for the final crystallization in the Examples.

Patent applications Nos. WO 2006/099433 and WO 2006/099468 (both by TEVA) describe preparation of duloxetine with high chemical and enantiomeric purity achieved by crystallization of crude duloxetine hydrochloride from water or organic solvents and their mixtures with water. Besides the above mentioned ethyl acetate and ethylmethylketone, the following solvents are claimed as suitable: acetone, methyl tert-butylether (MTBE), ethanol, isopropanol and n-butanol.
The crystallization makes use of the higher solubility of the purified substance at higher temperatures, typically at reflux, than at lower ones. This means that during crystallization the substance is exposed to heat stress, which can cause its partial decomposition. This may be manifested especially in large volumes where the heating and cooling has high requirements for time and energy. This method of crystallization may be very efficient from the point of view of the resulting purity, but on the other hand it may be very inefficient from the yield point of view, especially in the case of substances with a small difference in solubility at high and low temperatures.
Duloxetine hydrochloride belongs to the group of substances with such a small difference in solubility at high and low temperatures and moreover it is subject to decomposition when exposed to heat so that crystallization using heating-up to a high temperature of the solution is not a very suitable method of purification.

The present invention brings a very beneficial solution of preparation of highly pure duloxetine.

### Disclosure of Invention

The invention deals with a new method of purification of (*S*)-*N*-methyl-3-(1-naphtyloxy)-3-(2-thienyl) propylamine hydrochloride of formula I 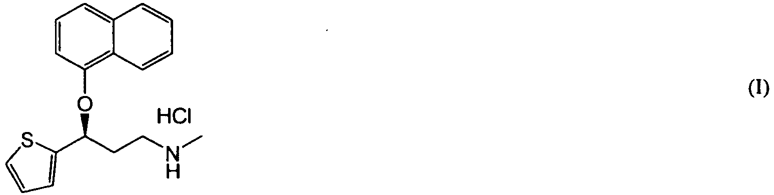 which comprises
(1) releasing the duloxetine base from its crystalline hydrochloride by the action of an organic or inorganic base in an aqueous environment; and
(2) transforming the duloxetine base to the hydrochloride by action of hydrochloric acid or gaseous hydrochloride in an organic solvent or a mixture of solvents. Crystalline hydrochloride is preferably obtained.

The duloxetine base is released from its hydrochloride in an aqueous environment by the action of an inorganic or organic base such as e.g. hydroxides and carbonates of alkali metals or ammonia or triethylamine. Preferably the release is carried out by the action of an aqueous solution of KOH, NaOH, K₂CO₃, Na₂CO₃ or ammonia.

The duloxetine base is extracted into a suitable organic solvent that is miscible with water to a limited extent. Preferably such solvent is selected from the group of ethers, esters and ketones.

The duloxetine base can also be prepared by demethylation of (*S*)-*N*-methylduloxetine or by reaction of 1-fluoronaphtalene with (*S*)-*N*-methyl-3-hydroxy-3-(2-thienyl)propanamine. The crude reaction mixture is usually diluted with water, pH of the aqueous layer is adjusted to the value of 7-12 if necessary and the duloxetine base is extracted into a suitable organic solvent that is partially miscible with water.

Suitable solvents for the extraction include e.g. methyl tert-butylether (MTBE), ethyl acetate (EtOAc), acetone, ethylmethylketone, methylisobutylketone, toluene, 2-methyltetrahydrofuran, and the like and their mutual mixtures.

Advantageously, for the release of 1 g of the base of substance I 2 ml of an aqueous solution of Na₂CO₃ are used. Preferably, 5 ml of the mixture of methyl tert-butyl ether and ethylmethylketone in the 4:1 proportion are used as the organic solvent for the extraction and subsequent transformation of the base of substance I to its hydrochloride.

The solution of the duloxetine base in an organic solvent is dried in a suitable way or is directly used for the preparation of duloxetine hydrochloride. This solution may accordingly contain a certain quantity of water in the range of 0 to roughly 5% by volume.

Solid duloxetine hydrochloride is prepared by neutralization reaction of the duloxetine base dissolved in an organic solvent or a mixture of organic solvents containing 0 to 5% of water with hydrochloric acid. For the preparation of duloxetine hydrochloride a 35% solution of hydrochloric acid in water is normally used. The yield and purity of duloxetin hydrochloride is considerably influenced by the used organic solvent or mixture of solvents and the content of water. Table 1 summarizes analyzes of chemical and optical purity together with the yields of duloxetine hydrochloride obtained by neutralization reaction of a 35% aqueous solution of hydrochloric acid and the duloxetine base prepared from 10 g of the initial crude duloxetine hydrochloride by the action of 20 of an aqueous solution of an inorganic base and subsequent extraction of the duloxetine base with 80 ml of an organic solvent. Chemical purity was evaluated with the use of HPLC, optical purity with the use of CE.

**Table 1. Influence of the conditions on the quality of the substance prepared from crude duloxetine hydrochloride by transformation, followed by neutralization reaction with concentrated hydrochloric acid**

| **Exp. No.** | **Conditions** (used inorganic base / organic solvent) | **3-isomer III** | **Sum of impurities** | **(*R*)-isomer II** | **Yield** |
|---|---|---|---|---|---|
| *Initial material* | - | *0.16 %* | *0.52%* | *0.30 %* | - |
| 1 | KOH / MTBE:MEK (6:1) | 0.09% | 0.36% | 0.25% | 62% |
| 2 | KOH / EtOAc | 0.09% | 0.40% | 0.20% | 33% |
| 3 | Na₂CO₃ / EtOAc:MTBE (6:1) | 0.09% | 0.13% | 0.12% | 63% |
| 4 | KOH / EtOAc:MEK (6:1) | 0.08% | 0.10% | 0.25% | 42% |
| 5 | NH₃ (aq.) / EtOAc:MTBE (5:1) | 0.07% | 0.16% | 0.11% | 72% |

Another aspect of this invention is a method of purification of (*S*)-*N*-methyl-3-(1-naphtyloxy)-3-(2-thienyl) propylamine hydrochloride of formula I, which comprises dissolution of this substance in a minimum quantity of methanol, containing 0 to 50% of water, and its transformation back into the solid phase (precipitation) by addition of a less polar solvent. Duloxetine hydrochloride is relatively well soluble, mainly at higher temperatures, in polar protic solvents such as e.g. water or lower alcohols or their mixtures. Duloxetine hydrochloride dissolves especially well in methanol. Its solubility in this solvent is 50 g/100 ml at 20 °C and over 70 g/100 ml at 50 °C. If the solution of duloxetine hydrochloride in methanol is diluted with another, less polar solvent (e.g. EtOAc, MEK, MTBE, etc., and their mutual mixtures), the solubility is considerably reduced and solid duloxetin hydrochloride is separated from the solution. Gradual addition of less polar solvents can proceed at the temperature of -20°C to 65 °C, preferably at -5 °C to 5 °C.
Preferably, 1 g of the hydrochloride of substance I is dissolved in 1 to 3 ml of methanol containing 0 to 50% of water at the temperature of 0 to 65 °C and the less polar solvent is gradually added at the temperature of -10 to 40 °C.

Methanol may contain a certain quantity of water, namely 0 to 45%, preferably 0 to 10% of water. Preferably, 1 g of the hydrochloride of substance I is dissolved in 2 ml of methanol containing less than 10% of water at the temperature of 25 °C and then 10 ml of the mixture of methyl tert-butyl ether and ethylmethylketone in the 1:1 proportion are slowly added at the temperature of 0 to 5 °C.

This process, i.e. precipitation of dissolved duloxetine hydrochloride from the solution by addition of another solvent, can be used for easy and efficient purification of this substance. The efficiency of this method is documented in table 2.
Initial crude duloxetine hydrochloride (10 g) was dissolved in 20 ml of methanol at 25 °C and subsequently precipitated in a crystalline form by addition of the specified organic solvent at the temperature of 0 to 5 °C.

**Table 2. Influence of the used organic solvent and water quantity for the quality of the substance prepared by precipitation of duloxetin hydrochloride from a solution in methanol by addition of a less polar solvent**

| **Exp. No.** | **Conditions** (used solvents) | **3-isomer III** | **Sum of impurities** | **(R)-isomer II** | **Yield** |
|---|---|---|---|---|---|
| *Initial material* | - | *0.16 %* | *0.52 %* | *0.30 %* | - |
| 1 | MeOH / MTBE (1:3) | 0.14 % | 0.18 % | 0.05 % | 93 % |
| 2 | MeOH / MEK (1:6) | 0.08 % | 0.07 % | 0.04 % | 81 % |
| 3 | MeOH / EtOAc (1:6) | 0.09 % | 0.16 % | 0.11 % | 49 % |
| 4 | MeOH / MEK / MTBE (1:3:3) | 0.09 % | 0.14 % | 0.09 % | 86 % |
| 5 | MeOH / water / MTBE (2:1:6) | 0.08 % | 0.15 % | 0.10 % | 62 % |

Advantages of this method as compared to conventional crystallization, i.e. hot dissolution of the substance (usually under reflux conditions) and gradual cooling of the solution accompanied by crystallization consist in low energy and material demands, ease and efficiency.

These purification methods of the substance of formula I mentioned above can be easily used in a large scale as well as it is not necessary to heat and subsequently cool large solution volumes. Decomposition of the dissolved substance is also limited at low temperatures.

The invention is described in a more detailed way in the following Examples.

### Examples

### Example 1:

(*S*)-*N*-methyl-3-(1-naphtyloxy)-3-(2-thienyl) propylamine hydrochloride (100 g) is stirred up in 10% aqueous sodium carbonate (200 ml) and the free duloxetine base is extracted with MTBE (2 x 200 ml). The solution is diluted with MEK (100 ml) and concentrated HCl is slowly added dropwise under stirring in such a quantity to achieve pH of the mother liquor of 3 to 5. Separated duloxetine hydrochloride is sucked off and washed with MTBE. The yield is 84 g (84 %).

### Example 2:

(*S*)-*N*-methyl-3-(1-naphtyloxy)-3-(2-thienyl) propylamine hydrochloride (100 g) is stirred up in 12% aqueous ammonia (200 ml) and the free duloxetine base is extracted with EtOAc (2 x 200 ml). The solution is diluted with MTBE (100 ml) and concentrated HCl is slowly added dropwise under stirring in such a quantity to achieve pH of the mother liquor of 3 to 5. Separated duloxetine hydrochloride is extracted and washed with MTBE. The yield is 75 g (75 %).

### Example 3:

(*S*)-*N*-methyl-3-(1-naphtyloxy)-3-(2-thienyl) propylamine hydrochloride (100 g) is dissolved in methanol (200 ml) at 40 °C. After cooling to 5 °C and inoculation MTBE (400 ml) is added dropwise during 1 hour. Separated duloxetine hydrochloride is sucked off and washed with MTBE. The yield is 95 g (95 %).

### Example 4:

(*S*)-*N*-methyl-3-(1-naphtyloxy)-3-(2-thienyl) propylamine hydrochloride (100 g) is dissolved in methanol (200 ml) at 40 °C. After cooling to 5 °C and inoculation a mixture of MTBE (200 ml) and MEK (200 ml) is added dropwise during 1 hour. Separated duloxetine hydrochloride is sucked off and washed with MTBE. The yield is 89 g (89 %).

### Survey of used analytic methods

### Chemical purity evaluation

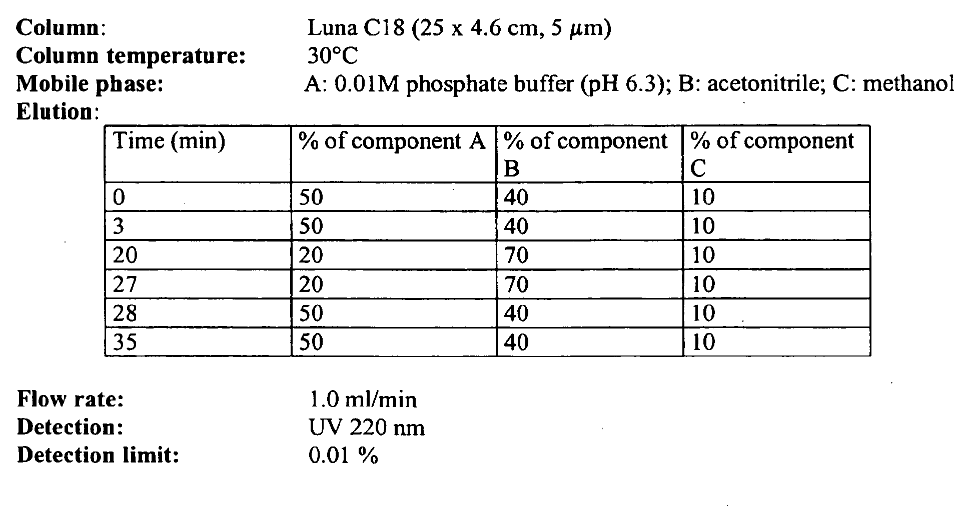

### Optical purity evaluation

| | |
|---|---|
| **Capillary:** | Agilent Technologies ,,extended light path capillary" (40 cm) |
| **Column temperature:** | 30 °C |
| **Basic electrolyte:** | 0.375 g of tris(hydroxymethyl)aminomethane and 300 µl of 85 % H₃PO₄ in 100 ml of water |
| **Separation electrolyte:** | 35 mg of HS-β-CD in 0.8 ml of basic electrolyte |
| **Voltage:** | - 15 kV |
| **Detection:** | UV 235 nm |
| **Detection limit:** | 0.04 % |

## Claims

1. A method of purification of (*S*)-*N*-methyl-3-(1-naphtyloxy)-3-(2-thienyl) propylamine hydrochloride of formula I, 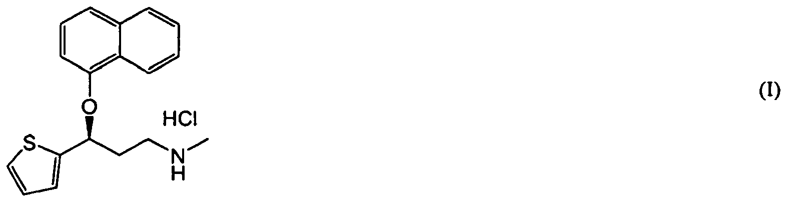 comprising dissolution of this substance in a minimum quantity of methanol containing 0 to 50% of water and its transformation back to the solid phase (precipitation) by addition of a less polar solvent.

2. The method in accordance with claim 1, **characterized in that** methyl tert-butyl ether, ethyl acetate, ethylmethylketone or their mutual mixtures are used as the less polar solvent.

3. The method in accordance with claim 1, **characterized in that** 1 g of the hydrochloride of substance I is dissolved in 1 to 3 ml of methanol containing 0 to 50% of water at the temperature of 0 to 65 °C and the less polar solvent is gradually added at the temperature of -10 to 40 °C.

4. The method in accordance with claim 1, **characterized in that** 1 g of the hydrochloride of substance I is dissolved in 2 ml of methanol containing less than 10% of water at the temperature of 25 °C and then 10 ml of the mixture of methyl tert-butyl ether and ethylmethylketone in the 1:1 proportion are slowly added at the temperature of 0 to 5 °C.

## Patentansprüche

1. Verfahren zur Reinigung von (*S*)-*N*-Methyl-3-(1-naphthyloxy)-3-(2-thienyl)propylamin-Hydrochlorid der Formel I, 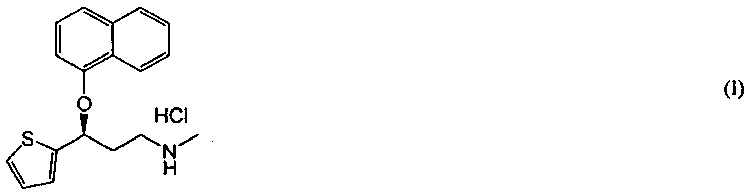 umfassend Lösen dieser Substanz in einer minimalen Menge Methanol, enthaltend 0 bis 50% von Wasser, und die Umwandlung zurück in die feste Phase (Fällung) durch Zugabe eines weniger polaren Lösungsmittels.

2. Verfahren nach Anspruch 1, **dadurch gekennzeichnet, dass** man Methyl-tert-butylether, Ethylacetat, Ethylmethylketon oder ihre gegenseitigen Mischungen als das weniger polare Lösungsmittel verwendet.

3. Verfahren nach Anspruch 1, **dadurch gekennzeichnet, dass** man 1 g des Hydrochlorids von Substanz I in 1 bis 3 ml Methanol, enthaltend 0 bis 50% Wasser, bei einer Temperatur von 0 bis 65 gelöst und das weniger polare Lösungsmittel allmählich bei einer Temperatur von -10 bis 40 °C zugibt.

4. Verfahren nach Anspruch 1, **dadurch gekennzeichnet, dass** man 1 g des Hydrochlorids von Substanz I in 2 ml Methanol, enthaltend weniger als 10% Wasser, bei einer Temperatur von 25 gelöst und dann 10 ml der Mischung aus Methyl-tert-butylether und Ethylmethylketon im Verhältnis 1:1 langsam bei einer Temperatur von 0 bis 5 °C zugibt.

## Revendications

1. Procédé de purification de (*S*)-*N*-méthyl-3-(1-naphtyloxy)-3-(2-thiényl)propylamine de formule I 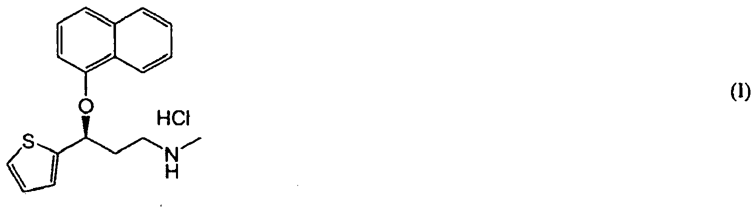 comprenant la dissolution de cette substance dans une quantité minimale de méthanol contenant de 0 à 50% de l'eau et sa transformation en arrière à la phase solide (précipitation) par addition d'un solvant moins polaire.

2. Procédé selon la revendication 1, **caractérisé en ce que** l'on utilise l'éther méthyl tert-butyle, l'acétate d'éthyle, éthylméthylcétone ou leurs mélanges mutuels comme le solvant moins polaire.

3. Procédé selon la revendication 1, **caractérisé en ce que** l'on dissout 1 g du chlorhydrate de la substance I dans 1 à 3 ml de méthanol contenant de 0 à 50% d'eau à la température de 0 à 65°C et le solvant moins polaire est ajouté progressivement à la température de -10 à 40°C.

4. Procédé selon la revendication 1, **caractérisé en ce que** l'on dissout 1 g du chlorhydrate de la substance I dans 2 ml de méthanol contenant moins de 10% d'eau à la température de 25°C et ensuite on ajoute lentement 10 ml du mélange d'éther de méthyle et de tert-butyle et éthylméthylcétone dans la proportion 1:1 à la température de 0 à 5°C.
